# EUROPEAN PATENT SPECIFICATION

(11) **EP 2 730 286 B1**
(45) Date of publication and mention of the grant of the patent: **20.06.2018**
(21) Application number: 12807575.1
(22) Date of filing: 03.07.2012
(51) Int. Cl.: A23L 21/20, A23L 33/105, A23L 33/10

(54) **COMPOSITION FOR MAINTAINING BONE HEALTH AND FOR TREATING OSTEOARTHRITIS AND OSTEOARTHROSIS OF THE JOINTS**
ZUSAMMENSETZUNG ZUR ERHALTUNG DER KNOCHENGESUNDHEIT UND ZUR BEHANDLUNG VON OSTEOARTHRITIS UND OSTEOARTHROSE DER GELENKE
COMPOSITION DESTINÉE À MAINTENIR LES OS EN BONNE SANTÉ ET À TRAITER L'OSTÉOARTHRITE OU L'OSTÉOARTHROSE DES ARTICULATIONS

(30) Priority: 05.07.2011 RU 2011127494
(43) Date of publication of application: 14.05.2014
(73) Proprietor: OBSCHESTVO S ORGANICHENNOI OTVESTVENNOSTJU "PARAFARM", Penza 440026 (RU)
(72) Inventor: TRIFONOV, Vyacheslav Nikolaevich, Zarechnij 442960 Penzenskaja obl. (RU); ELISTRATOVA, Julia Anatoljevna, Penza 440026 (RU); ELISTRATOV, Konstantin Gennadievich, Penza 440061 (RU); KURUS, Natalia Vyacheslavovna, Penza 440061 (RU); HOMYKOVA, Irina Vladimirovna, Penza 440003 (RU); ELISTRATOVA, Tatiana Viktorovna, Penza 440018 (RU)
(74) Representative: Engel, Christoph Klaus
(86) International application number: PCT/RU2012/000528
(87) International publication number: WO 2013/006094

(56) References cited:
- EP-A1- 2 135 517
- WO-A1-01/01996
- RU-C1- 2 412 616
- RU-C1- 2 412 616
- RU-C2- 2 314 717
- 'Biologicheski aktivnaya dobavka ''Epam-31 ''.' FEDERALNY REESTR BIOLOGICHESKI AKTIVNYKH DOBAVOK K PISCHE. MOSCOW, IZDATELSTVO «KOGELET» 2000, page 300, XP008172983

## Description

The invention relates to a composition for improving functional status of the locomotor system and is a modern, efficient means for controlling joint diseases. The invention further relates to the use of the composition in the manufacture of a nutrition product, additive or drug, and to a method for stimulating cartilage tissue restoration, said method comprising ingesting an effective amount of the composition. The composition according to the present invention can be used in the manufacture of a nutrition product, additive, treat or drug intended for a human or domestic animals, promotes the formation of cartilage in mammals and the prevention of osteoarthritis in domestic animals or a human, thereby enhancing an individual's activity or mobility. According to another aspect, the invention relates to the use of a plant or plant extract selected on the basis of its ability to stimulate bone morphogenic proteins and/or inhibit bone resorption in the preparation of a composition intended for maintaining bone health in mammals and for preventing, relieving and/or treating bone diseases. In addition, the invention provides a method for preventing, relieving and/or treating bone diseases or for maintaining bone health which increases efficacy of the treatment, and for preventing the development of complications and side effects, said method comprising the administration of an effective amount of the composition. The invention further relates to a method for treating, relieving and/or prophylaxis of osteoarthritis in domestic animals or a human, the method comprising a step of feeding an individual with the composition.

Centuries-old medical practice indicates that almost everyone has occasional backache and limb joint pain (R. Nordemar. Back Pain. - Moscow: Meditsina, 1988 - 144 pp. in total / 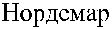 P. 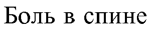 . - M.: 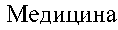 , 1988 - 144 c). Rheumatoid arthritis, which is the most severe form of an inflammatory joint disease, occurs in 3% of the world's population, while non-inflammatory (degenerative) changes (i.e., arthrosis) occur in about 10% of the world's population (V.P. Kessel. When Joints Ache, - Moscow: Znaniye, 1979. - 96 pp. in total / 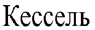 B.Π. 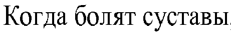 , - M.: 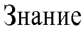 , 1979. - 96 c).

Currently, various medicinal products (urodan, aspirin, reopirine diclofenac, analgin, antibiotics etc.) are employed for the treatment of joint diseases, said products being of insufficient efficacy. More than 100 nonspecific pharmaceuticals for the therapy of joint diseases are known; however, it is still early to speak of a pharmaceutical inflation, since this problem is far from being solved due to insufficient therapeutic efficacy and poor long-term effects.

Osteoarthritis (OA) is a progressive chronic disease mainly affecting joints which are mostly physically stressed, such as hip and knee joints. Such conditions involve the affection of an entire joint, resulting from series of destructive and **regenerative** processes, which have an inevitable impact on joint anatomy and functioning, thereby affecting all joint components, such as cartilages, subchondral bones and synovial **tissues.** OA is thus a result of interrelation between systemic factors (e.g., age or obesity) and local factors (e.g., traumas or high stress), which, in their turn, are modulated by a number of triggering factors, possibly in combination with an infection or inflammatory processes of various etiologies. Previously, the cartilage was considered to be responsible for the incidence of OA and as the only target organ. However, a great amount of data indicating that subchondral bones may act as a pathogenic factor responsible for OA onset and development has been obtained recently.

Taking into account these events together, efforts to treat the condition by eliminating individual causal processes and using local or systemic anti-inflammatory drugs, or intraarticular injections of hyaluronic acid, were made over many years. Later on, use of preparations impacting on bone metabolism, such as diphosphonates, was suggested. Some drugs, described in a rather informal way, such as cartilage protectors (anthraquinones, chondroitin sulfate and the like), are also of a limited use, though their actual therapeutic ability is very low and inconsistent.

It has been established that morphological changes of the structure of a subchondral bone precede joint cartilage lesions. Abnormalities in the microcirculation in subchondral joint regions are characterized with the obstruction of venous outflow accompanied with the increased intraosseous pressure, which causes an excruciating pain syndrome.

A remedy having immunomodulatory, antiviral, antibacterial, antioxidant, membrane stabilizing effects and stimulating **cartilage** and connective **tissue** regeneration, the remedy comprising substances belonging to the group of cartilage protectors, i.e. chondroitin sulfate and glucosamine, as well as antibacterial components and excipients with adjuvants is known (Patent No. 2349339, RU. Published on March 20, 2009).. A remedy for treating joint diseases, comprising chondroitin sulfate, dimethyl sulfoxide, a preservative, an emulgel base, is known (Patent No. 2353349, RU. Published on April 27, 2009). A remedy for treating joint diseases, comprising a glucosamine salt, a salt of chondroitin sulfate and dimethyl sulfoxide, is known (Patent No. 2239438, RU. Published on March 20, 2006, and Patent No. 6528, EA - analog. Published on February 24, 2006). A remedy for the treatment and prophylaxys "Glucosamine Sulphate" used for the treatment and prophylaxis of the locomotor system is known (Biologically Active Food Supplements. - Moscow: Nauka, 2002, p. 47 / 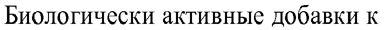 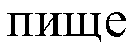 . - M.: Hayκa, 2002, c. 47). The "Glucosamine sulphate" preparation comprises glucosamine. Glucosamine has a cartilage protecting effect. Entering a human body, it is delivered towards a cartilage and other **tissues**, where it is converted into chondroitin sulfate. Chondroitin sulfate serves as a major source of structural material for **connective tissue.** It is involved in formation of joint cartilage, ligaments, tendons, presents in vessel and bronchi walls, skin, and mucous membranes. Chondroitin sulfate is an efficient means for regeneration of the cartilage tissue and formation of the synovial fluid, for reducing muscle fatigability, as well as for reinforcing any connective **tissue** in a human body. However, "Glucosamine sulphate" is a monodrug. A remedy named "Sustavite" (" 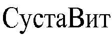 "), comprising a combined composition for the treatment and prophylaxis, wherein in addition to a glucosamine derivative, chondroitin sulfate, medicinal plants, vitamins, calcium and enzymes are contained, is known (Biologically Active Food Supplements. - Moscow: Nauka, 2002, p. 49 / 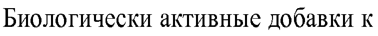 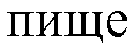 . - M.: Hayκa, 2002, c. 49). The known composition provides a nutritional support for the osteo-articular system, promotes connective **tissue** growth and regeneration and stimulates the production of collagen and substances required for cartilage growth; and it represents the closest prior art of the present invention.

EP 2 135 517 A1 shows a food composition intended for the prevention, alleviation and/or treatment of bone disorders and maintenance of bone health in humans and pets. The composition comprises as an active ingredient an effective amount of at least one plant or plant extract containing phytochemicals having the ability to induce bone morphogenic protein expression.

EP 2 508 083 A1 describes a biologically active food supplement for the prevention of osteoporosis comprising a calcium compound and drone brood.

The drawbacks of the known remedies consist in that they lack an active substance which allows increasing tonicity of venous system, improving venous drainage, thereby attenuating the feeling of heaviness and swelling in legs which is associated with joint diseases.

The objective of the present invention consists in providing a composition of active substances acting effectively upon eliminating a pain syndrome in a joint.

The objective of the present invention consists in providing a composition providing the osteoarticular system with the most effective nutritional support as well as connective **tissue** growth and regeneration without a side effect.

The objective of the present invention consists in a possible collagen synthesis for regeneration of the connective **tissue, cartilage tissue** with retaining the anti-inflammatory and analgesic effect.

The set objective is achieved by that the composition comprises:
A complex of natural substances which is based on:
**Plants**, producing factors of cartilage tissue regeneration (glycosides, i.e. taraxacine, taraxacerine which are real cartilage protectors; Vitamin PP). **The pharmacological effect of glycosides** should, on one hand, provide **chondrocytes propagation** and **change the state of the cartilage matrix,** on the other hand. **Vitamin PP** (nicotinic acid, vitamin B3, niacin, vitamin PP) belongs to the group of B vitamins and is involved in redox processes, protein metabolism, carbohydrates metabolism, cholesterol metabolism, iron metabolism and represents the only vitamin taking part in hormonal status establishment. Vitamin PP plays a crucial role in the synthesis of sex hormones (estrogens, progesterone, testosterone) as well as hormones produced by adrenal cortex (cortisone), thyroid gland (thyroxin) and pancreas (insulin). Cortisone, thyroxin and insulin actively influence cartilage regeneration and formation of chondrocytes of the tissue. The release of somatotropin causes the synthesis of insulin-like growth factor (IGF-1) in the liver, which appears to be the strongest anabolic for the regeneration in bones, bone marrow, cartilage and synovial fluid.

**A drone brood**, which is a donator of androgenic steroids: prolactin, estradiol, progesterone, testosterone. Of amino acids, i.e., glycine, alanine and others, having a stimulating effect on CHONDROCYTE (cartilage cells) formation and SYNOVIAL fluid replenishment.

**A phytochemical compound**, which restores metabolic processes, enhances capillary permeability, improves bone tissue blood supply, thereby allowing a cartilage to regenerate and repair its structure.

### Basic concepts.

**A healthy joint, as well as an organism, consists of living cells. Bones, cartilage, meniscus and other joint tissues are comprised of living cells. Only living cells are capable of regeneration and complete restoration, especially leg joints, are considered to be difficult to cure. To cope with a joint disease, whether it is arthrosis, arthritis, polyarthritis, meniscal detachment, it is necessary to know what causes the disease to become chronic.**

Joints are known to be formed by bone articular surfaces coated with cartilage tissue. Upon various movements, the cartilage functions as a shock-absorber, reducing pressure on articulating bone surfaces and providing their smooth sliding relative to each other.

The cartilage responds flexibly to stereotypical movements, repeated thousands of times: pushes while walking or running, jumping, and the like. This repetitive stress results in aging and disruption of a part of the fibers. A healthy joint compensates these losses by producing the same amount of new fibers. Joint and spine diseases arise upon imbalance between synthesis of new building material required for cartilage tissue regeneration, and disruption thereof.

This finding has allowed to take a fresh look at the joint disease problem and to associate it with metabolic diseases. Moreover, it was proved that this is the latter which actually represents **primary** causes, while the changes in the osteo-articular system accompanied with subsequent diseases represent **secondary** causes. This also explains why joint diseases at initial stages develop latently, without visible symptoms. Targeted regulation of metabolism not only brings joint deformation to stop, regardless of a stage of a disease, but also promotes regeneration of the morphologic structure of the cartilage.

Cartilage tissue represents a type of the connective tissue acting in the organism as a body support. A **perichondrium** providing nutrition and growth of the cartilage is an indispensable attribute of the cartilage, except for a joint cartilage. The cartilage in joints is "bare" and contacts directly with the articular internal milieu, i.e., **synovial fluid**. It acts as a sort of lubrication between sliding joint surfaces covered with a smooth hyaline cartilage. Bone and spine cartilages constantly experience both static and dynamic stresses.

Cartilage cells (**chondrocytes**) and extracellular **matrix** consisting of fibers and a ground substance are its main components. Moreover, the cartilage is actually composed mostly of intercellular substance. A small number of cells in the cartilage, these cells being surrounded by a great amount of **extracellular space**, i.e., **the matrix,** is a distinctive feature of the cartilage compared to other types of body tissues. Poor cartilage post-injury recovery is determined by the small number of cells capable of propagation, so that its repair (restoration) occurs mainly due to the extracellular matrix. The extent of the cartilage low metabolic activity will be evident from the following comparison. The liver protein composition renews completely in 4(!) days.

**The renewal of the cartilage collagen is only 50% in 10 (!) years.** Accordingly, any cartilage tissue trauma is, understandably, almost incurable, **unless special measures directed to increase the number of chondrocytes which will form a new matrix are undertaken.**

The joint cartilage contains plenty of water (femoral head cartilage of a young man contains 75 g water per 100 grams of the tissue). **Hyaluronic acid** promotes the matrix to bind water, thereby providing elastic and flexural properties of the tissue.

Sources for the regeneration are represented by the following: 1) **the cartilage per se**; 2) joint **synovial membrane**, growing over from the edges of the defect and transforming into a cartilage-like tissue; 3) **bone cells**, which are, notably, derived from the cartilage and can transform "back" into a tissue which structurally resembles the cartilage, where necessary; 4) **bone marrow cells**, which may serve as a source for regeneration in deep cartilage injuries combined with bone injuries.

Bone blood flow is quite intensive, with bone tissue having 5% of cardiac blood output. Blood flow velocity is only 2-fold lower than in brain. Bone tissue fate, formation, and disruption thereof depend on blood supply, series of hyperemic, congestive and anemic conditions. Bone blood flow is similar to the skeletal muscle blood flow; however, taking into consideration that bones are to a large extent composed of an inorganic substance (combined calcium salts), blood flow per 1 bone cell of the cell mass is higher THAN in the muscle. In a certain sense, bones have a higher metabolic activity and blood supply thereof measured on active cell weight basis is more than 10-fold higher than the muscle blood supply. (G. I. Lavrishcheva et al. "Bone Blood Supply and Regeneration" Kishinev, "Shtinnitsa", 1981 / 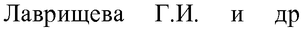 . « 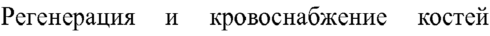 » 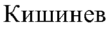 , « 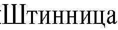 », 1981 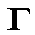 ). Thus, any bone blood supply abnormalities result in dystrophic changes of bones, bone marrow, cartilage, synovial fluid. On the contrary, improved blood supply due to increased capillary permeability enhances regeneration in bones, bone marrow, cartilage, synovial fluid.

Composition according to the present invention:
The pharmacological effect should, on one hand, induce chondrocytes propagation, and, on the other hand, modify the state of the cartilage matrix and restore the collagen skeleton surrounding bones and cartilage tissue, improve blood supply, enhance regeneration in bones, bone marrow, cartilage, synovial fluid.

A composition for preventing, treating and relieving course of a connective tissue disease consists of at least one component of each of the three following groups:
**1) Plants:** The composition comprises a PLANT intended for preventing, relieving and/or treating bone diseases and maintaining bone health in a human and domestic animals, the composition comprising as an active ingredient an effective amount of at least one plant (or plant extract) or more plants (or plant extracts), selected from the group consisting of: burdock, dandelion, marsh cinquefoil, birch, Hypericum, goldenrod, Urtica, mint, Glycyrrhiza, Potentilla alba, Potentilla erecta, dogrose, Polemonium caeruleum, Valeriana, corn, Cucumaria, milk thistle, oats, agrimony, immortelle, ginseng, sage, Stellaria, gourd, Salix, strawberries, chicory, Elytrigia, sunroot, bilberry, calendula, horseradish, garlic, aspen, birdweed (knot-grass), plantains Inula, redstem buckwheat, chamomile flowers, melissa, tormentil, blue cornflower, cottonweed, bur beggar-ticks. As used herein, "a plant" means any part thereof, i.e., leaves, tubers, fruits, seeds, roots, grains, embryos or cell cultures. A plant or plant extract can be either a lyophilized extract from leaves, roots and/or fruits, depending on a species of the plant being used, or freshly harvested plant, or enriched fraction obtained via an extraction method using an inorganic or organic solvent, the method being known in the art.
**2) Drone brood** can be the following substance: drone larvae, or lyophilized drone larvae extract, or enriched fraction obtained via an extraction method using an inorganic or organic solvent, the method being known in the art, or a combination thereof.
**3) A phytochemical compound**, i.e. a substance containing one or more substances selected from the following group: quercetine, dihydroquercetine, rutin.

The composition according to the present invention may be used in the manufacture of a food composition. Said composition may be in the form of either a nutrient-balanced food product or feed for domestic animals or nutrition additive, treat or pharmaceutical formulation.

The composition may be used either separately or in combination with other plants, such as, for example, chicory, tea, cocoa, or in combination with other bioactive molecules, such as antioxidants, fatty acids, prebiotic fibers, glucosamine, chondroitin sulfate.

In one embodiment, a food composition for human consumption is prepared. Said composition can be a fully nutrient-balanced formulation, dairy product, cooled or long-life beverage, soup, nutrition additive, meal replacement within daily diet and a nutritional bar or confectionery.

The nutritional formulation is preferably intended for enteral administration, for example, as a powder, tablet, capsule, liquid concentrate, solid product or ready-to-drink beverage. To make a dry powder nutritional formulation, a homogenized mixture is passed through a suitable dryer, for example, a spray dryer or freeze dryer, thereby obtaining a powder.

In another embodiment, the nutritional composition includes milk-based cereal product mixed with a prebiotic formulation. The milk-based cereal product preferably is a baby food product, which serves as a vehicle for the prebiotic formulation.

In the further embodiment, a regular food product, for example, fermented dairy product, yoghurt, fromage frais, renneted curd, confectionery, sweet or sweetened beverage, candy bar, cereal flakes or breakfast cereal bars, beverages, dehydrated milk, soy based products, non-dairy fermented products or nutrition additives for meal in clinics, may be enriched with at least one plant or plant extract of the invention.

The amount of a plant or plant extract in the composition may vary, depending on a plant species and a part thereof being used. In the preferred embodiment, an effective daily dose is at least about 1 mg, more preferably 1 mg to 200 mg of active molecules.

In one embodiment, a pharmaceutical composition comprising the compound as indicated above, in an amount sufficient for achieving a desired effect in an individual may be prepared. Said composition may be in the form of a tablet, liquid, capsules, soft capsules, paste or lozenges, gum or drinkable solutions or emulsions, dry oral additive, wet oral additive. The pharmaceutical composition will also comprise vehicles and excipients suitable for delivering corresponding active molecules of various nature to a target **tissue**. Type of a vehicle/excipient and an amount thereof will depend on a nature of a substance and intended drug delivery method and/or route of administration. It is apparent that those skilled in the art are able to select suitable constituents and galenic forms, relying on their experience and knowledge.

The nutrient-balanced feed composition for domestic animals according to the present invention may be formulated as a powder, a dry form, either as a treat or wet, cooled or shelf-stable product for domestic animals. This can be a cooled product or long shelf life product. The aforementioned products for domestic animals can be prepared using any known method.

Where necessary, the feed composition for domestic animals may also comprise a prebiotic, probiotic microorganisms or another active agent, e.g., a long-chain fatty acid. Preferably, the amount of a prebiotic in the feed compositions for domestic animals is less than 10 mass%. By way of example, a prebiotic may constitute about 0.1 to 5 mass% of the feed weight. Where the feed comprises chicory as a prebiotic source, the chicory may be added so that the amount thereof in the feed mixture is about 0.5 to 10 mass%, more preferably about 1 to 5 mass%.

In cases where a probiotic microorganism is used, 1 gram of the feed for domestic animals in the preferred embodiment comprises about 10⁴ to 10¹⁰ cells of the probiotic microorganism, in the more preferred embodiment about 10⁶ to 10⁸ cells of the probiotic microorganism. The feed for domestic animals may contain about 0.5 to 20 mass% of the mixture of probiotic microorganisms, preferably about 1 to 6 mass%, for example, about 3 to 6 mass%.

Mineral substances and vitamins are added into the feed for domestic animals, where necessary, so that it is fully nutrient-balanced. In addition, other ingredients, such as sugar, salt, spices, herbs, flavorings and the like may be introduced into the feed for domestic animals, if required.

In another embodiment, nutrition additives improving the quality of feeds for domestic animals may be prepared. These nutrition additives may be encapsulated or formulated as a powder, and packed together with a basal feed or separately, the latter being either wet or dry. By way of example, a powder comprising the extracts of the present invention may be packed in sachets either as a powder, or in the form of a gel or lipid, or any other suitable vehicle. These separately packed additives may be further provided either together with the basal feed, or in multiple packages for later use together with the basal feed or treat, according to a user instruction.

A feed amount to be consumed by a domestic animal for achieving a desired effect will depend on size, species and age of the domestic animal. However, the amount of feed for domestic animals, the amount providing a daily dose of about 0.5 to 5 g of dried plants per 1 kg of an animal body weight will be normally adequate for dogs and cats.

Administering the food composition or feed composition as described above to a human or domestic animal results in improved bone regeneration during the treatment of fractures. The composition is able to stimulate bone formation, increase bone mineral density in the growth stage and optimize peak bone mass. In particular, it may provide optimal bone growth in children. The aforesaid food composition helps to prevent bone loss, in particular age-related bone loss in mammals or bone loss associated with long-term hospitalization. It reduces risk of osteoporosis and improves post-fracture **recovery**. In addition, it promotes cartilage formation in mammals, prevents osteoarthritis in a human and domestic animals, which results in enhanced activity or mobility of an individual.

The following examples are provided only for illustration of the present invention and they are not intended to limit the scope of the present invention.

### Preparative examples for the product.

### Example 1

Ingredients:
- Dihydroquercetine powder, 10 kg
- drone brood lyophilizate, 10 kg
- ground dandelion root powder, 10 kg. The mixture is stirred to homogeneity, the resulting product is then encapsulated.

### Example 2

Ingredients:
- Dihydroquercetine powder, 100 kg
- drone brood lyophilizate, 400 kg
- ground dandelion root powder, 500 kg. The mixture is stirred to homogeneity, and then tableted.

For carrying out the experiments, a formulation comprising per 1 tablet: dihydroquercetine 100 mg, drone brood lyophilizate 100 mg, powder dandelion root 200mg, was selected. Dosing regimen: cyclic, 1 tablet 2 times a day for 30 days followed by a gap of 14 days. The treatment duration is 9 months.

The method for treating provided herein has been applied at "Sekrety dolgoletiya" (« 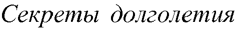 ») Medical Center, in Penza city, in 30 patients suffering from lower limbs osteoarthrosis, complicated with synovitis, periarthritis, chondro- and osteomatosis, varicose disease, osteoporosis. Age 54 to 72 years. Duration of the disease 1-10 years.

Patients were found to experience pain in legs, joint stiffness, restriction of joint motion, reduced quality of life. Physical examination revealed swelling in the knee joint area, supra-acetabular region of the hip joints, deformation and defiguration of the knee joints, increased skin temperature in the area of knee and hip joints, and restriction of motion therein.

The treatment according to the method of the invention resulted in the improvement in all the patients, i.e. the pain in the knee and hip joints reduced or eliminated, range of motion therein increased, the skin temperature decreased according to thermometry data. Rheovasography and volumetric flow rate of muscle blood showed improving in blood supply in the affected limb region, that formation of subchondral cysts did not occur or was delayed; patients' quality of life improved, the necessity of endoprosthetic surgery was put off.

The above method for treating patients with osteoarthrosis, suffering from associated diseases, relieves inflammation, improves tissue trophicity and **cartilage** metabolism and periarticular tissue metabolism, normalizes circulation, has a positive impact on subchondral bone remodeling in the affected limb region. The improvement occurred after the first cycle of the administration of the composition. There was an increase in the motor activity and relief of pain syndrome, which results in sustained remission, improved quality of life for the patients and putting off the necessity of endoprosthetic surgery.

Accordingly, said method for treating improves the quality of patients' life, helps to prevent the disease development and disability of the patients suffering from the I-II initial stages of osteoarthrosis, and to put off the necessity of endoprosthetic surgery in the patients with the III-IV stages of the disease.

## Claims

1. A composition consisting of at least one component of each of the three following groups (a), (b) and (c):
(a) a plant selected from the following group: burdock, dandelion, marsh cinquefoil, birch, Hypericum, goldenrod, Urtica, mint, Glycyrrhiza, Potentilla alba, Potentilla erecta, dogrose, Polemonium caeruleum, Valeriana, corn, Cucumaria, milk thistle, oats, agrimony, immortelle, ginseng, sage, Stellaria, gourd, Salix, strawberries, chicory, Elytrigia, sunroot, bilberry, calendula, horseradish, garlic, aspen, birdweed (knotgrass), plantain, Inula, redstem buckwheat, chamomile flowers, melissa, tormentil, blue cornflower, cottonweed, bur beggar-ticks;
(b) drone brood;
(c) one or more substances selected from the following group: quercetine, dihydroquercetine, rutin.

2. The composition according to claim 1, wherein the plant implies any portion thereof, such as leaves, tubers, fruits, seeds, roots, grains, embryos or cell cultures.

3. The composition according to claim 1, in the form of a nutrient-balanced food product or feed for domestic animals, nutrition additive, or pharmaceutical composition.

4. The composition according to claims 1 to 3 for use as a medicament in preventing, treating or relieving a connective tissue disease.

5. The composition for use according to claim 4 wherein the composition is administered separately or in combination with other plants, such as, for example, chicory, tea, cocoa, or other bioactive molecules, such as antioxidants, fatty acids, prebiotic fibers, glucosamine, chondroitin sulfate.

6. The composition according to claim 1 for use as a medicament in treating, relieving or preventing bone diseases or maintaining bone health..

7. The composition according to claim 1 for use as a medicament in increasing bone formation, bone mineral density in the growth stage and optimizing peak bone mass in human or domestic animals.

8. The composition according to claim 1 for use as a medicament in treating, relieving and/or prophylaxis of osteoarthritis in human or domestic animals.

9. The composition according to claim 1 for use as a medicament in treating or preventing osteoporosis.

10. The composition according to claim 1 for use as a medicament in stimulating bone regeneration during the treatment of fractures.

11. The composition according to claim 1 for use as a medicament in for reducing bone loss, in a human or domestic animals.

12. The composition for use as a medicament according to the preceding claim, wherein the bone loss is age-related bone loss.

## Patentansprüche

1. Zusammensetzung, bestehend aus wenigstens einer Komponente aus jeder der drei folgenden Gruppen (a), (b) und (c):
(a) eine Pflanze, ausgewählt aus der folgenden Gruppe: Große Klette, Löwenzahn, Sumpfblutauge, Birke, Johanniskraut, Goldraute, Brennnessel, Minze, Süßholz, Potentilla alba, Potentilla erecta, Hundsrose, Polemonium caeruleum, Baldrian, Mais, Cucumaria, Mariendistel, Hafer, Odermenning, Strohblume, Ginseng, Salbei, Sternmiere, Flaschenkürbis, Weide, Erdbeere, Chicoree, Quecke, Topinambur, Blaubeere, Ringelblume, Meerrettich, Knoblauch, Espe, Vogelknöterich (Weggras), Wegerich, Alant, rotstieliger Buchweizen, Kamillenblüten, Melisse, Blutwurz, Blaue Kornblume, Ruhrkraut, Acker-Zweizahn;
(b) Drohnenbrut;
(c) eine oder mehrere Substanzen, ausgewählt aus der folgenden Gruppe: Quercetin, Dihydroquercetin, Rutin.

2. Zusammensetzung nach Anspruch 1, wobei die Pflanze jeglichen Teil davon beinhaltet, wie Blätter, Wurzelknollen, Früchte, Samen, Wurzeln, Körner, Embryonen oder Zellkulturen.

3. Zusammensetzung nach Anspruch 1 in Form eines nährstoffausgewogenen Nahrungsprodukts oder Futtermittels für Haustiere, Nahrungsergänzungsmittels oder einer pharmazeutischen Zusammensetzung.

4. Zusammensetzung nach den Ansprüchen 1 bis 3 zur Verwendung als Arzneimittel bei der Vorbeugung, Behandlung oder Linderung einer Bindegewebskrankheit.

5. Zusammensetzung zur Verwendung nach Anspruch 4, wobei die Zusammensetzung getrennt oder in Kombination mit anderen Pflanzen wie zum Beispiel Chicoree, Tee, Kakao oder anderen bioaktiven Molekülen, wie Antioxidantien, Fettsäuren, präbiotischen Fasern, Glucosamin, Chondroitinsulfat, verabreicht wird.

6. Zusammensetzung nach Anspruch 1 zur Verwendung als Arzneimittel bei der Behandlung, Linderung oder Vorbeugung von Knochenkrankheiten oder bei der Aufrechterhaltung der Knochengesundheit.

7. Zusammensetzung nach Anspruch 1 zur Verwendung als Arzneimittel bei der Erhöhung der Knochenbildung, der Knochenmineraldichte im Wachstumsstadium und der Optimierung der maximalen Knochenmasse bei einem Menschen oder bei Haustieren.

8. Zusammensetzung nach Anspruch 1 zur Verwendung als Arzneimittel bei der Behandlung, Linderung und/oder Prophylaxe von Osteoarthritis bei einem Menschen oder bei Haustieren.

9. Zusammensetzung nach Anspruch 1 zur Verwendung als Arzneimittel bei der Behandlung oder Vorbeugung von Osteoporose.

10. Zusammensetzung nach Anspruch 1 zur Verwendung als Arzneimittel bei der Stimulierung der Knochenregeneration während der Behandlung von Brüchen.

11. Zusammensetzung nach Anspruch 1 zur Verwendung als Arzneimittel bei der Reduktion von Knochenverlust bei einem Menschen oder bei Haustieren.

12. Zusammensetzung zur Verwendung als Arzneimittel nach dem vorhergehenden Anspruch, wobei der Knochenverlust altersbedingter Knochenverlust ist.

## Revendications

1. Composition constituée d'au moins un composant de chacun des trois groupes (a), (b) et (c) suivants :
(a) une plante sélectionnée dans le groupe suivant : la bardane, le pissenlit, la potentille des marais, le bouleau, le millepertuis, la solidage, l'Urtica, la menthe, la réglisse, Potentilla alba, Potentilla erecta, la rose de chien, Polemonium caeruleum, la valériane, le maïs, Cucumaria, le chardon Marie, l'avoine, l'aigremoine, l'immortelle, le ginseng, la sauge, la stellaire, la coloquinte, le saule, le fraisier, la chicorée, l'élytrigie, le topinambour, la myrtille, le calendula, le raifort, l'ail, le tremble, la renouée des oiseaux, le plantain, l'aunée, Eriogonum rubricaule, les fleurs de camomille, la mélisse, la tormentille, le bleuet, l'anémone cylindrique, le bident à feuilles tripartites ;
(b) le couvain de mâles ;
(c) une ou plusieurs substances sélectionnées dans le groupe suivant : quercétine, dihydroquercétine, rutine.

2. Composition selon la revendication 1, dans laquelle la plante implique une partie quelconque de celle-ci, par exemple les feuilles, les tubercules, les fruits, les semences, les racines, les graines, les embryons ou les cultures cellulaires.

3. Composition selon la revendication 1, sous la forme d'un produit alimentaire ou d'un aliment pour animaux domestiques équilibré en éléments nutritifs, d'un additif nutritionnel, ou d'une composition pharmaceutique.

4. Composition selon les revendications 1 à 3 pour son utilisation en tant que médicament dans la prévention, le traitement ou le soulagement d'une maladie du tissu conjonctif.

5. Composition pour son utilisation selon la revendication 4 dans laquelle la composition est administrée séparément ou en combinaison avec d'autres plantes, telles que, par exemple, la chicorée, le thé, le cacao, ou d'autres molécules bioactives, telles que des antioxydants, des acides gras, des fibres prébiotiques, de la glucosamine, de la chondroïtine sulfate.

6. Composition selon la revendication 1 pour son utilisation en tant que médicament dans le traitement, le soulagement ou la prévention de maladies osseuses ou la conservation de la santé osseuse.

7. Composition selon la revendication 1 pour son utilisation en tant que médicament dans l'augmentation de la formation osseuse, de la densité minérale osseuse durant le stade de croissance et dans l'optimisation de la masse osseuse maximale chez les êtres humains ou les animaux domestiques.

8. Composition selon la revendication 1 pour son utilisation en tant que médicament dans le traitement, le soulagement et/ou la prophylaxie de l'arthrose chez les êtres humains ou les animaux domestiques.

9. Composition selon la revendication 1 pour son utilisation en tant que médicament dans le traitement ou la prévention de l'ostéoporose.

10. Composition selon la revendication 1 pour son utilisation en tant que médicament dans la stimulation de la régénération osseuse durant le traitement des fractures.

11. Composition selon la revendication 1 pour son utilisation en tant que médicament pour la réduction de la perte osseuse, chez un être humain ou des animaux domestiques.

12. Composition pour son utilisation en tant que médicaments selon la revendication précédente, dans laquelle la perte osseuse est une perte osseuse liée à l'âge.
